# EUROPÄISCHE PATENTSCHRIFT

(11) **EP 1 957 125 B1**
(45) Veröffentlichungstag und Bekanntmachung des Hinweises auf die Patenterteilung: **30.03.2011**
(21) Anmeldenummer: 06805713.2
(22) Anmeldetag: 14.09.2006
(51) Int. Cl.: A61L 27/22, A61F 2/06, A61F 2/04, A61F 2/02

(54) **VERFAHREN ZUR HERSTELLUNG EINES HOHLPROFILS AUF BASIS EINES VERNETZTEN, GELATINE ENTHALTENDEN MATERIALS SOWIE IMPLANTATE IN FORM VON HOHLPROFILEN**
METHOD FOR PRODUCING A HOLLOW PROFILE USING A CROSS-LINKED, GELATINOUS MATERIAL AND IMPLANTS IN THE FORM OF HOLLOW PROFILES
PROCEDE POUR PRODUIRE UN PROFILE CREUX AU MOYEN D'UN MATERIAU RETICULE ET GELATINEUX, ET IMPLANTS SE PRESENTANT SOUS LA FORME DE PROFILES CREUX

(30) Priorität: 17.11.2005 DE 102005054943
(43) Veröffentlichungstag der Anmeldung: 20.08.2008
(73) Patentinhaber: GELITA AG, 69412 Eberbach (DE)
(72) Erfinder: AHLERS, Michael, 69412 Eberbach (DE)
(74) Vertreter: Hoeger, Stellrecht & Partner Patentanwälte
(86) Internationale Anmeldenummer: PCT/EP2006/008948
(87) Internationale Veröffentlichungsnummer: WO 2007/057067

(56) Entgegenhaltungen:
- WO-A-2005/111121
- DE-A1- 2 938 438
- US-A- 5 716 660
- CHEN Y-S ET AL: "An in vivo evaluation of a biodegradable genipin-cross-linked gelatin peripheral nerve guide conduit material" BIOMATERIALS, ELSEVIER SCIENCE PUBLISHERS BV., BARKING, GB, Bd. 26, Nr. 18, Juni 2005 (2005-06), Seiten 3911-3918, XP004697270 ISSN: 0142-9612

## Beschreibung

Die vorliegende Erfindung betrifft ein Verfahren zur Herstellung eines Hohlprofils auf Basis eines vernetzten, Gelatine enthaltenden Materials.

Die Erfindung betrifft ferner Implantate in Form von Hohlprofilen.

Implantate in Form von Hohlprofilen, d.h. insbesondere röhrenförmige Implantate, finden in verschiedenen Bereichen der Medizin Anwendung. Ein wichtiges Anwendungsgebiet betrifft dabei den Einsatz solcher Implantate, um das Lumen von röhrenförmigen Organen oder Geweben aufrechtzuerhalten und ein Kollabieren von Gefäßwänden zu verhindern. Derartige Implantate werden auch als Stents bezeichnet und kommen beispielsweise in Blutgefäßen, dem Darm und der Speiseröhre zum Einsatz.

In anderen Fällen werden Hohlprofile implantiert, um eine Funktion als Drainage zu erfüllen, d.h. um beispielsweise Gewebeflüssigkeit aus einem entzündeten Bereich abzuleiten.

Die oben beschriebenen Implantate werden zum Teil aus dauerhaften Materialien wie z.B. Edelstahl hergestellt und müssen dann wieder entfernt werden, wenn ihre Funktion nicht mehr benötigt wird. Um dem Patienten diesen weiteren (z.T. operativen) Eingriff zu ersparen, sind daher Implantate aus resorbierbaren Biopolymeren wie beispielsweise Gelatine, Kollagen oder Chitosan, die vom Körper nach einer gewissen Zeit abgebaut werden, eine wichtige Alternative.

In der Offenlegungsschrift DE 29 38 438 A1 wird eine flexible Sonde zur inneren Schienung von Hohlorganen wie z.B. des Darms offenbart. Die Sonde ist in dem Höhlorgan resorbierbar und besteht aus mit einem Vernetzungsmittel vernetzter Gelatine und/oder vernetztem Kollagen und einem physiologisch unbedenklichen Weichmacher.

Das US-Patent 5,716,660 beschreibt ein Verfahren zur Herstellung einer PTFE-Prothese, die ein Biomaterial enthält. Dabei werden die Hohlräume eines Substrates aus expandiertem PTFE mit einer Flüssigkeit gefüllt, die ein lösliches biokompatibles Material enthält, welches insbesondere durch eine Vernetzung verfestigt werden kann. Das biokompatible, bioabbaubare Material ist ausgewählt aus Kollagen, Gelatine sowie Derivaten und Mischungen hiervon.

In dem Artikel "An in vivo evaluation of a biodegradeable genipin-cross-linked gelatin peripheral nerve guide conduit material" von Chen et al. (Biomaterials 26 (2005) 3911-3918) wird eine bioabbaubare Nervenleitschiene beschrieben, die aus mit Genipin vernetzter Gelatine hergestellt ist. Die Herstellung erfolgt ausgehend von einer Gelatinelösung, die auf einem Dorn getrocknet wird. Anschließend wird die Nervenleitschiene durch Einwirkung einer wässrigen Genipinlösung vernetzt.

Die Aufgabe der vorliegenden Erfindung besteht darin, ein Herstellungsverfahren für resorbierbare Hohlprofile vorzuschlagen, das einfach durchzuführen ist und bei dem die Eigenschaften des erzeugten Hohlprofils über einen weiten Bereich beeinflusst werden können.

Diese Aufgabe wird erfindungsgemäß gelöst durch ein Verfahren zur Herstellung eines Implantats in form eines Hohlprofils auf Basis eines vernetzten, Gelatine enthaltenden Materials, wobei das Hohlprofil einen polygonalen Querschnitt aufweist und eine Wandung umfasst, welche ein Lumen umgibt, umfassend:
a) Herstellen einer wässrigen Lösung eines Gelatine enthaltenden Materials;
b) partielles Vernetzten des gelösten, Gelatine enthaltenden Materials;
c) Aufbringen der Lösung auf die Oberfläche eines das Lumen definierenden Formelements;
d) mindestens partielles Trocknenlassen der Lösung auf dem Formelement unter Ausbildung eines Hohlprofils auf Basis des vernetzten, Gelatine enthaltenden Materials ; und
e) Vernetzen des in dem Hohlprofil enthaltenen Materials.

Unter einem polygonalen Querschnitt wird im Zusammenhang mit der vorliegenden Erfindung jeder Querschnitt des Hohlprofils verstanden, der eine endliche oder unendliche Anzahl von Ecken aufweist, d.h. insbesondere auch ovale, elliptische oder runde Querschnitte. Hohlprofile mit einem runden Querschnitt, d.h. zylindrische Hohlprofile, stellen im Rahmen der vorliegenden Erfindung den einfachsten Fall und die wohl am häufigsten benötigte Form dar.

Die erfindungsgemäße Herstellung des Hohlprofils mit Hilfe eines das Lumen definierenden Formelements hat gegenüber anderen Herstellungsmethoden deutliche Vorteile. Sie ist mit geringem technischem Aufwand zu realisieren, wobei die Abmessungen des Hohlprofils sowie dessen Querschnittsform über die Wahl des Formelements einfach variiert und zugleich mit hoher Genauigkeit vorgegeben werden können.

Eine Extrusion des Hohlprofils wäre mit einem deutlich höheren Aufwand verbunden. Zudem stellt sich hier das Problem, das bei der erfindungsgemäßen Verwendung von Gelatine als Ausgangsmaterial, auf deren Vorteile weiter unten eingegangen wird, die Extrusion meist nur unter solchen Temperaturbedingungen durchgeführt werden kann, die zu einem partiellen thermischen Abbau der Gelatine führen.

Eine weitere Möglichkeit zur Herstellung von Hohlprofilen wäre das Aufrollen eines Flächenmaterials, wie z.B. einer Folie. Dies führt jedoch unweigerlich zu inhomogenen Materialeigenschaften des Hohlprofils zumindest entlang einer Nahtstelle; ferner kann diese Methode bei sehr kleinen Durchmessern des Hohlprofils kaum durchgeführt werden.

Das Aufbringen der Lösung auf die Oberfläche des Formelements erfolgt bei einer bevorzugten Ausführungsform der Erfindung durch Eintauchen des Formelements in die Lösung. Dabei wird ein sehr gleichmäßiger Auftrag der Lösung auf das Formelement gewährleistet, was zu einem Hohlprofil mit einer im Wesentlichen gleichmäßig dicken Wandung führt. Nach dem zumindest partiellen Trocknenlassen kann das Hohlprofil von dem Formelement abgezogen werden.

Alternativ besteht die Möglichkeit, die Lösung des Gelatine enthaltenden Materials auf das Formelement aufzusprühen. Allerdings ist damit ein nicht ganz so gleichmäßiger Auftrag ohne besondere Vorkehrungen möglich.

Das Formelement sollte bevorzugt aus einem inerten Material mit einer glatten Oberfläche, wie z.B. Edelstahl, gebildet sein. Um das anschließende Abziehen des Hohlprofils zu erleichtern, kann das Formelement vor dem Aufbringen der Lösung mit einem Trennmittel (z.B. Wachs) behandelt werden.

Während die Abmessungen des Lumens, d.h. insbesondere der Innendurchmesser des Hohlprofils, durch das das Lumen definierende Formelement vorgegeben werden kann, ist die Dicke der Wandung von der Menge der aufgebrachten Lösung abhängig. Diese Menge kann wiederum, insbesondere beim Eintauchen des Formelements, in erster Linie über die Viskosität der Lösung gesteuert werden.

Die Wandstärke des Hohlprofils kann noch weiter erhöht werden, wenn c) und d) im Anschluss an d) ein- oder mehrmals wiederholt werden. Insbesondere betrifft dies ein mehrmaliges Eintauchen des Formelements in die Lösung, wobei jedes Mal eine gleichmäßige Materialschicht aufgebracht wird.

Nachdem zunächst das Aufbringen der Lösung des Gelatine enthaltenden Materials auf das Formelement beschrieben wurde, wird im Folgenden noch im Einzelnen auf dessen Zusammensetzung sowie die Vernetzung einzugehen sein, welche erfindungsgemäß vor dem Aufbringen der Lösung auf das Formelement geschehen muss.

Für die Herstellung von unter physiologischen Bedingungen unlöslichen, jedoch resorbierbaren Hohlprofilen ist der erfindungsgemäße Einsatz von Gelatine äußerst vorteilhaft, da diese vom Körper rückstandslos resorbiert werden kann. Im Gegensatz zu dem mit Gelatine verwandten Material Kollagen ist Gelatine in hoher Reinheit und mit reproduzierbarer Zusammensetzung erhältlich und ist frei von immunogenen Telopeptiden, die Abwehrreaktionen des Körpers auslösen können.

Um eine optimale Bioverträglichkeit des hergestellten Hohlprofils bei der medizinischen Anwendung zu gewährleisten, enthält das Material bevorzugt eine Gelatine mit einem besonders geringen Gehalt an Endotoxinen. Bei Endotoxinen handelt sich um Stoffwechselprodukte oder Bruchstücke von Mikroorganismen, welche in dem tierischen Rohmaterial vorkommen. Der Endotoxingehalt von Gelatine wird in internationalen Einheiten pro Gramm (I.E./g) angegeben und gemäß dem LAL-Test bestimmt, dessen Durchführung in der vierten Ausgabe des Europäischen Arzneibuches (Ph. Eur. 4) beschrieben ist.

Um den Gehalt an Endotoxinen möglichst gering zu halten, ist es vorteilhaft, die Mikroorganismen möglichst frühzeitig im Zuge der Gelatineherstellung abzutöten. Ferner sollten entsprechende Hygienestandards beim Herstellungsprozess eingehalten werden.

Somit kann der Endotoxingehalt von Gelatine durch bestimmte Maßnahmen beim Herstellungsprozess drastisch gesenkt werden. Zu diesen Maßnahmen zählen in erster Linie die Verwendung frischer Rohmaterialien (z.B. Schweineschwarte) unter Vermeidung von Lagerzeiten, die sorgfältige Reinigung der gesamten Produktionsanlage unmittelbar vor Beginn der Gelatineherstellung sowie gegebenenfalls das Auswechseln von Ionenaustauschern und Filtersystemen in der Produktionsanlage.

Die im Rahmen der vorliegenden Erfindung eingesetzte Gelatine weist bevorzugt einen Endotoxingehalt von 1.200 I.E./g oder weniger, noch mehr bevorzugt von 200 I.E/g oder weniger auf. Optimalerweise liegt der Endotoxingehalt bei 50 I.E./g oder weniger, jeweils gemäß dem LAL-Test bestimmt. Im Vergleich hierzu weisen manche handelsübliche Gelatinen Endotoxingehalte von über 20.000 I.E./g auf.

Das Gelatine enthaltende Material, auf dessen Basis das Hohlprofil hergestellt wird, ist bevorzugt zu einem überwiegenden Teil aus Gelatine gebildet. Darunter fallen insbesondere Gelatineanteile von 60 Gew.% oder mehr, bevorzugt von 75 Gew.% oder mehr. Neben Gelatine kann das Material beispielsweise noch weitere Biopolymere wie etwa Alginate oder Hyaluronsäure enthalten, um das Eigenschaftsprofil der Hohlprofils noch spezifischer an eine bestimmte Applikation anzupassen.

Bei einer bevorzugten Ausführungsform der Erfindung umfasst das Gelatine enthaltende Material zusätzlich einen Weichmacher. Durch einen solchen Zusatz kann die Flexibilität des hergestellten Hohlprofils in trockenem Zustand deutlich erhöht werden. Dies kann von Vorteil sein, wenn beispielsweise eine hohe Biegeelastizität des Hohlkörpers beim Implantieren erwünscht ist. Als Weichmacher sind z.B. Glycerin, Oligoglycerine, Oligoglykole und Sorbit geeignet, wobei Glycerin am meisten bevorzugt ist.

Die gewünschte Flexibilität des Hohlprofils kann über die Menge des Weichmachers gesteuert werden. Bevorzugt liegt der Anteil an Weichmacher in dem Material bei 12 bis 40 Gew.%. Besonders vorteilhaft sind dabei Anteile von 16 bis 25 Gew.%. Die angegebenen Gewichtsprozente beziehen sich hierbei auf die Gesamtmasse aller Komponenten des Gelatine enthaltenden Materials, die sowohl in der Lösung gemäß a) als auch in dem hergestellten Hohlprofil vorliegen.

Bei einer weiteren Ausführungsformen des erfindungsgemäßen Verfahrens, insbesondere wenn kein Zusatz von Weichmacher erfolgt, ist das Material im Wesentlichen vollständig aus Gelatine gebildet.

Die Gelatinekonzentration in der wässrigen Lösung gemäß a) liegt bevorzugt bei 5 bis 45 Gew.%, am meisten bevorzugt bei 10 bis 30 Gew.%. Konzentrationen in diesem Bereich sind insbesondere für ein Eintauchen des Formelements in die Lösung geeignet.

Erfindungsgemäß wird das Gelatine enthaltende Material gemäß b) partiell vernetzt, wobei vorzugsweise die Gelatine selbst vernetzt wird. Da Gelatine an sich wasserlöslich ist, ist diese Vernetzung erforderlich, um ein zu schnelles Auflösen des Hohlprofils zu verhindern und eine ausreichende Lebensdauer unter physiologischen Bedingungen zu gewährleisten.

Dabei bietet Gelatine den zusätzlichen Vorteil, dass die Resorptionsgeschwindigkeit des vernetzten Materials, bzw. die Zeitdauer bis zur vollständigen Resorption, durch die Wahl des Vernetzungsgrades über einen weiten Bereich eingestellt werden kann.

Das erfindungsgemäße Verfahren umfasst ferner ein Vernetzen (e) des in dem Hohlprofil enthaltenen Materials. Bevorzugt wird bei dieser weiteren Vernetzung insbesondere die Gelatine vernetzt.

Der Vorteil einer solchen zweistufigen Vernetzung besteht grundsätzlich darin, dass ein hoher Vernetzungsgrad und damit einhergehend lange Abbauzeiten erzielt werden können. Dies kann mit einem einstufigen Verfahren unter Erhöhung der Konzentration an Vernetzungsmittel nicht in gleichem Maße verwirklicht werden, da durch ein zu starkes Vernetzen des gelösten Materials dieses nicht mehr verarbeitet und auf die Oberfläche des Formelements aufgebracht werden kann.

Andererseits ist auch eine Verwendung eines unvernetzten Materials und dessen Vernetzen ausschließlich nach der Herstellung des Hohlprofils nicht geeignet, da dieser hierbei an den von außen zugänglichen Grenzflächen stärker vernetzt als in den inneren Bereichen des Hohlprofils, was sich in einem inhomogenen Abbauverhalten widerspiegelt.

Die zweite Vernetzung gemäß e) kann durch Einwirken einer wässrigen Lösung eines Vernetzungsmittels durchgeführt werden, bevorzugt ist jedoch das Einwirken eines gasförmigen Vernetzungsmittels.

Die Vernetzung kann sowohl chemisch als auch enzymatisch durchgeführt werden.

Bevorzugte chemische Vernetzungsmittel sind Aldehyde, Dialdehyde, Isocyanate, Carbodiimide und Alkyldihalogenide. Besonders bevorzugt ist dabei Formaldehyd, insbesondere für die zweite Vernetzung in der Gasphase, wodurch gleichzeitig eine Sterilisierung des Hohlprofis bewirkt wird.

Als enzymatisches Vernetzungsmittel wird bevorzugt das Enzym Transglutaminase eingesetzt, welches eine Verknüpfung der Gluatamin- und Lysinseitenketten von Proteinen, insbesondere auch von Gelatine, bewirkt.

Die erfindungsgemäß hergestellten Hohlprofile können zum Teil erstaunlich lange Lebensdauern unter physiologischen Bedingungen aufweisen, wobei diese durch den Grad der Vernetzung sehr gezielt eingestellt werden können. Ein Maß hierfür bietet die Resorptionsstabilität unter physiologischen Standardbedingungen. So können Hohlprofile hergestellt werden, die unter physiologischen Standardbedingungen beispielsweise länger als eine Woche, länger als zwei Wochen oder länger als vier Wochen stabil bleiben.

Der Begriff der Stabilität ist hier dahingehend zu verstehen, dass das Hohlprofil seine ursprüngliche Form sowohl bei Lagerung in trockenem Zustand als auch während der angegebenen Zeitdauer bei physiologischen Standardbedingungen (PBS-Puffer, pH 7,2, 37 °C) im Wesentlichen erhält und erst anschließend strukturell in erheblichem Umfang hydrolytisch abgebaut wird. Die physiologischen Bedingungen, denen die Hohlprofile bei einer Verwendung als Implantate ausgesetzt sind, sind in erster Linie durch Temperatur, pH-Wert und Ionenstärke gekennzeichnet und können *in vitro* durch eine Inkubation unter den genannten Standardbedingungen simuliert werden.

Überraschenderweise können auch Hohlprofile mit einem hohen Vernetzungsgrad hergestellt werden, die durch die Zugabe eines Weichmachers dennoch über eine relativ hohe Flexibilität verfügen, d.h. Lebensdauer und Flexibilität können in einem gewissen Bereich unabhängig voneinander gesteuert werden.

Bei einer weiteren Ausführungsform des erfindungsgemäßen Verfahrens wird das Hohlprofil im Anschluss an d) in Längsrichtung verstreckt. Eine solche Verstreckung kann sich in verschiedener Hinsicht positiv auswirken. Zum einen führt die zumindest partielle Ausrichtung der Gelatinemoleküle entlang einer Vorzugsrichtung zu verbesserten mechanischen Eigenschaften des Hohlprofils, d.h. zu einer Erhöhung der Reißfestigkeit und/oder der Reißdehnung, insbesondere in Längsrichtung. Dies kann in bestimmten Anwendungsbereichen von großem Vorteil sein.

Ferner wird durch das Verstrecken die Herstellung von Hohlprofilen mit einem sehr kleinen Innendurchmesser ermöglicht, da die Längsverstreckung zu einer radialen Kontraktion des Hohlprofils führt. Entsprechend kleine Durchmesser, die allein mittels eines Lumen definierenden Formelements kaum realisiert werden können, sind beispielsweise bei Hohlprofilen für Nervenleitschienen für das periphere Nervensystem gefordert.

Besonders gut lässt sich das Hohlprofil verstrecken, wenn das Gelatine enthaltende Material einen Weichmacher umfasst.

Bevorzugt wird das Hohlprofil unmittelbar vor dem Verstrecken durch eine Erhöhung der Temperatur und/oder des Wassergehalts in einen thermoplastischen Zustand überführt. Dies kann beispielsweise dadurch erfolgen, dass das Hohlprofil helßern Wasserdampf ausgesetzt wird. Die Verstreckung des Hohlprofils wird vorteilhafterweise mit einem Verstreckungsverhältnis von 1,4 bis 8 durchgeführt, wobei ein Verstreckungsverhältnis von bis zu 4 bevorzugt ist.

Bei einer weiteren Ausführungsform des erfindungsgemäßen Verfahrens wird das Hohlprofil vor dem Verstrecken bis zu vier Wochen gelagert , bevorzugt bei Raumtemperatur. Dadurch kann die Reißfestigkeit der erfindungsgemäß hergestellten Hohlprofile weiter erhöht werden. Deutliche Effekte sind dabei bereits nach einer Lagerzeit von etwa drei Tagen zu beobachten, während ab einer Lagerzeit von etwa sieben Tagen in der Regel keine wesentliche Steigerung mehr erzielt werden kann.

Die zweite Vernetzung (e) erfolgt vorzugsweise nach einem eventuellen Verstrecken des Hohlprofils. Dies ist deshalb vorteilhaft, weil die Moleküle in dem partiell vernetzten Material dann noch über eine ausreichende Bewegungsfreiheit verfügen und sich so zumindest teilweise entlang einer Vorzugsrichtung orientieren können und in diesem orientierten Zustand bleiben.

Bei einer weiteren Ausführungsform des erfindungsgemäßen Verfahrens erfolgt eine Zugabe eines Verstärkungsstoffes zu der Lösung gemäß a). Auf diese Weise können verstärkte Hohlprofile mit einer erhöhten mechanischen Festigkeit hergestellt werden. Insbesondere kann dadurch einem Ausreißen des Hohlprofils beim chirurgischen Vernähen entgegengewirkt werden. Die Verstärkungsstoffe sollen physiologisch verträglich und am besten ebenfalls resorbierbar sein.

Je nach Wahl des Verstärkungsstoffes lässt sich neben der Beeinflussung der mechanischen Eigenschaften auch die Stabilität gegen Resorptionsmechanismen in gewissem Umfang beeinflussen. Insbesondere lässt sich die Resorptionsstabilität der Verstärkungsstoffe unabhängig von den Komponenten des Gelatine enthaltenden Materials des Hohlprofils wählen.

Die Verstärkungsstoffe zeigen schon bei Anteilen von 5 Gew.% eine merkliche Verbesserung der mechanischen Eigenschaften des Hohlprofils. Die Gewichtsanteile beziehen sich dabei auf die gesamte Trockenmasse in der Lösung gemäß a), d.h. alle Bestandteile der Lösung mit Ausnahme von Wasser.

Oberhalb von 60 Gew.% lässt sich in der Regel keine signifikante Verbesserung mehr erreichen und/oder die gewünschten Resorptionseigenschaften oder auch die notwendige Flexibilität des Hohlprofils lassen sich nur noch schwierig darstellen.

Die Verstärkungsstoffe lassen sich aus partikulären und molekularen Verstärkungsstoffen sowie Mischungen hiervon auswählen.

Bei den partikulären Verstärkungsstoffen empfiehlt sich insbesondere die Verwendung von Verstärkungsfasern. Die Fasern sind dabei bevorzugt ausgewählt aus Polysaccharid- und Proteinfasern, insbesondere Kollagenfasern, Seide und Baumwollfasern, sowie aus Polylactidfasern und Mischungen hiervon.

Andererseits sind molekulare Verstärkungsstoffe ebenso geeignet, um die mechanischen Eigenschaften und, falls gewünscht, auch die Resorptionsstabilität des Hohlprofils zu verbessern.

Bevorzugte molekulare Verstärkungsstoffe sind Insbesondere Palylactidpolymere und deren Derivate, Cellulosederivate und Chitosan und dessen Derivate. Auch lassen sich die molekularen Verstärkungsstoffe als Mischungen einsetzen.

Die Aufgabe der Erfindung liegt ferner darin, ein resorbierbares Implantat in Form eines Hohlprofils zur Verfügung zu stellen, das vielfältig einsetzbar ist und dessen Eigenschaften an die jeweiligen Anforderungen angepasst werden können.

Die Aufgabe wird erfindungsgemäß gelöst durch ein Implantat in Form eines Hohlprofils, das auf der Basis eines vernetzten, Gelatine enthaltenden Materials hergestellt ist, wobei das Hohlprofil einen polygonalen Querschnitt aufweist und eine Wandung umfasst, welche ein Lumen umgibt, wobei das Hohlprofil gemäß dem oben beschriebenen Verfahren hergestellt ist.

Eine Vielzahl möglicher Modifikationen eines solchen Hohlkörpers zur Anpassung seiner Eigenschaften an die spezifischen Erfordernisse einer Applikation wurden bereits weiter oben im Zusammenhang mit dem erfindungsgemäßen Verfahren beschrieben.

Die besonderen Vorteile, die durch die Verwendung eines vernetzten, Gelatine enthaltenen Materials erzielt werden können, wurden ebenfalls bereits im Zusammenhang mit dem erfindungsgemäßen Verfahren beschrieben.

Eine bevorzugte Ausführungsform des erfindungsgemäßen Implantats betrifft einen Stent für die Speiseröhre. Derartige Stents kommen bei einer Verengung der Speiseröhre (Ösophagusstenose) zum Einsatz, die sowohl angeboren als auch erworben sein kann (z.B. durch Verätzung). Bei der angeborenen Ösophagusstenose, die in erster Linie bei Kindern auftritt, liegt eine Fehlbildung der Muskulatur der Speiseröhre vor, so dass diese mittels eines Stents offen gehalten werden muss.

Bei Verwendung von herkömmlichen Speiseröhrenstents z.B. aus Edelstahl, müssen diese nach einer bestimmten Zeit wieder entfernt werden, was für den Patienten eine zusätzliche Belastung darstellt und das Risiko von Verletzungen birgt. Demgegenüber bieten die erfindungsgemäßen resorbierbaren Stents den Vorteil, dass sie nach einer bestimmten, vorgegebenen Zeit vom Körper abgebaut werden, wenn sie nicht mehr benötigt werden oder wenn ein neuer Stent eingesetzt werden muss. Letzteres ist z.B. bei Kindern auf Grund des Wachstums der Fall.

Durch den vorgebbaren Vernetzungsgrad des Gelatine enthaltenden Materials, wie oben beschrieben, kann die Abbauzeit des Stents so eingestellt werden, wie dies für die jeweilige Behandlungssituation erforderlich ist. Typischerweise liegt die Abbauzelt des Stents bei ein bis zwei Wochen.

Eine weiterer bevorzugter Anwendungsbereich der erfindungsgemäßen Implantate betrifft Stents für den Darm. Diese werden insbesondere bei chirurgischen Eingriffen in den betroffenen Darmabschnitt eingesetzt, um bei einer auftretenden Undichtigkeit das Entweichen des Darminhalts zu verhindern. Gleichzeitig wird beispielsweise eine Naht (oder eine entzündete oder erkrankte Stelle) geschützt und kann an der Außenseite des Stents heilen. Auch hier erweist sich die vorgebbare Abbauzeit des Stents als besonderer Vorteil.

Ein weiterer möglicher Anwendungsbereich des erfindungsgemäßen Implantats ist ein Stent für die Luftröhre.

Die oben beschriebenen Stents weisen bevorzugt einen Innendurchmesser von 5 bis 30 mm auf, wobei Innendurchmesser von 8 bis 20 mm für die meisten Anwendungsfälle besonders bevorzugt sind, und eine mittlere Wandstärke von 300 bis 1.500 µm.

Abhängig von der jeweiligen Applikation kann bei dem erfindungsgemäßen Implantat das Gelatine enthaltende Material in einem inneren, dem Lumen benachbarten Bereich der Wandung einen anderen Vernetzungsgrad aufweisen auf als in einem äußeren Bereich. Im Fall von Stents für den Darm oder die Speiseröhre kann es z.B. bevorzugt sein, wenn der Vernetzungsgrad im äußeren Bereich etwas niedriger ist. Dadurch kann das Hohlprofil von außen in einem gewissen Umfang vergelen und somit an der Speiseröhre bzw. am Darm anhaften. Von Innen her bleibt der Stent hingegen möglichst fest und bietet damit gute Gleltelgenschaften.

Die erfindungsgemäßen Implantate können neben den oben angesprochenen Bereichen auch als Stents für weitere Organe bzw. Gewebe, insbesondere für Blutgefäße, Harnleiter, Harnröhre, Eileiter und Gallenblasengang eingesetzt werden.

In vielen dieser Fällen kann das Implantat bzw. der Stent auch als ein temporärer Gewebeersatz und/oder als Matrix für eine Regeneration des Gewebes dienen. Bei einer solchen Applikation werden die Enden eines durchtrennten Blutgefäßes (oder eines anderen oben erwähnten Gewebes) von beiden Seiten in das Implantat eingeführt und somit verbunden. Die Hohlröhre kann dabei als eine Matrix wirken, die ein Wachstum und eine Regeneration des Gewebes fördert. Gleichzeitig stellt das Implantat eine seitliche Barriere für Fremdzellen dar, die die Heilung .eventuell stören würden. Die Abbauzeit des Implantats kann wiederum auf die benötigte Regenerationszeit des Gewebes abgestimmt werden.

Einen besonderen Anwendungsbereich erfindungsgemäßer Leitstrukturen stellen Nervenleitschienen dar, welche zur Regeneration durchtrennter Nervenfasern (Axone) eingesetzt werden. Hierfür müssen die Hohlprofile einen sehr kleinen Innendurchmesser aufweisen, bevorzugt im Bereich von 800 bis 1.200 µm.

Eine weitere Ausführungsform des erfindungsgemäßen Implantats betrifft schließlich eine Drainage. Solche Drainagen werden unter anderem eingesetzt, um das Abfließen von Flüssigkeit (z.B. Blut oder Gewebeflüssigkeit) aus einem entzündeten oder verletzten Bereich zu ermöglichen. Die flexibel wählbaren Durchmesser und Abbauzeiten der erfindungsgemäßen Implantate ermöglichen einen Einsatz als Drainage in den verschiedensten Bereichen des Körpers.

Das erfindungsgemäße Implantat kann insbesondere auch als Drainage für das Auge eingesetzt werden. Solche Drainagen, bei denen es sich um sehr dünne Röhrchen handelt, werden zur Ableitung von Kammerwasser aus dem Augapfel im Fall von Augenüberdruck (Glaukom) eingesetzt.

Die erfindungsgemäße .Drainage für das Auge weist bevorzugt einen Innendurchmesser von 50 bis 200 µm auf.

Erfindungsgemäße Implantate mit kleinen Durchmessern, wie sie z.B. für Nervenleitschienen und Drainagen für das Auge benötigt werden, können insbesondere mit Hilfe dem erfindungsgemäßen,Verfahrens hergestellt werden, indem die Hohlröhre wie oben beschrieben verstreckt wird.

Diese und weitere Vorteile der Erfindung werden anhand der nachfolgenden Beispiele unter Bezugnahme auf die Figuren näher erläutert. Es zeigen im Einzelnen:
- Figur 1:: fotografische Darstellung erfindungsgemäßer Implantate; und
- Figur 2:: lichtmikroskopische Aufnahme eines erfindungsgemäßen Implantats im Querschnitt.

### Beispiel 1: Herstellung eines erfindungsgemäßen Implantats

Dieses Beispiel betrifft die Herstellung eines Implantats in Form eines Hohlprofils mit einem Innendurchmesser von 1 cm mittels des erfindungsgemäßen Verfahrens.

Hierfür wurden zunächst 130 g Schweineschwartengelatine (Bloomstärke 300 g) in einer Mischung aus 468 g Wasser und 52 g Glycerin als Weichmacher bei 60 °C gelöst und die Lösung mittels Ultraschall entgast. Dies entspricht einem Anteil an Weichmacher in dem Material von ca. 29 Gew.%, bezogen auf die Masse von Gelatine und Glycerin.

Nach Zugabe von 6,5 g einer wässrigen, 2,0 Gew.%igen Formaldehydlösung (1000 ppm Vernetzer bezogen auf die Gelatine) wurde die Lösung homogenisiert, nochmals entgast und die Oberfläche von Schaum befreit. Dann wurde ein Edelstahldorn als Formelement mit einem Durchmesser von 1 cm, der zuvor mit einem Trennwachs eingesprüht worden war, in einer Länge von ca. 10 cm kurz in die Lösung eingetaucht. Nach dem Herausziehen des Dorns aus der Lösung wurden dieser gedreht, so dass die anhaftende Lösung eine möglichst gleichmäßige Schicht bildete.

Nach etwa eintägigem Trocknen bei 25 °C und einer relativen Luftfeuchtigkeit von 30% wurde das gebildete Hohlprofil von dem Dorn abgezogen. Das auf diese Weise hergestellte Implantat mit einem Innendurchmesser von 1 cm kann beispielsweise als Stent für die Speiseröhre eingesetzt werden.

Um die physiologische Abbauzeit des Implantats zu verlängern, wurde die darin enthaltene Gelatine einer weiteren Vernetzung unterzogen. Hierzu wurde das Implantat in einem Exsikkator für 17 Stunden dem Gleichgewichtsdampfdruck einer 17 Gew.%igen wässrigen Formaldehydlösung bei Raumtemperatur ausgesetzt.

Ein Implantat mit einem höheren Vernetzungsgrad im inneren Bereich kann in diesem Fall z.B. dadurch erhalten werden, dass der Formaldehyddampf ausschließlich durch das Lumen des Hohlprofils geleitet wird. Alternativ können unterschiedliche Vernetzungsgrade auch so realisiert werden, dass der Dorn nacheinander in Lösungen mit verschiedenen Konzentrationen an Vernetzungsmittel eingetaucht wird.

Es versteht sich, dass die Eigenschaften des hier beschriebenen Implantats in vielfältiger Weise modifiziert werden können, indem insbesondere die Größe und Gestalt des Dorns bzw. des Formelements, die Anteile an Gelatine, Weichmacher und Vernetzungsmittel in der Lösung, die Anzahl der Tauchgänge, und die Intensität der Nachvernetzung an die jeweiligen Erfordernisse angepasst werden.

### Beispiel 2: Herstellung weiterer erfindungsgemäßer Implantate

Dieses Beispiel betrifft die Herstellung von Implantaten in Form von Hohlprofilen mit kleinen Innendurchmessern von etwa 2.000 µm, 1.100 µm bzw. 150 µm mittels des erfindungsgemäßen Verfahrens.

Es wurde eine Lösung aus 100 g Schweineschwartengelatine (Bloom-Stärke 300 g) in einer Mischung aus 260 g Wasser und 40 g Glycerin als Weichmacher hergestellt, wie in Beispiel 1 beschrieben. Dies entspricht einem Anteil an Weichmacher in dem Material von ca. 29 Gew.%, bezogen auf die Masse von Gelatine und Glycerin.

Nach Zugabe von 4 g einer wässrigen, 2,0 Gew.%igen Formaldehydlösung (800 ppm Vernetzer bezogen auf die Gelatine) wurde die Lösung homogenisiert, nochmals entgast und die Oberfläche von Schaum befreit. Eine Reihe von Edelstahlpins mit einem Durchmesser von 2 mm, die zuvor mit einem Trennwachs eingesprüht worden waren, wurden in einer Länge von ca. 3 cm kurz in die Lösung eingetaucht. Nach dem Herausziehen der Pins aus der Lösung wurden diese senkrecht gehalten, so dass die anhaftende Lösung eine möglichst gleichmäßige Schicht bildete.

Nach etwa eintägigem Trocknen bei 25 °C und einer relativen Luftfeuchtigkeit von 30% konnten die gebildeten Hohlprofile (Röhrchen) von den Edelstahlpins abgezogen werden. Sie wiesen einen Innendurchmesser von 2.000 µm und eine mittlere Wandstärke von ca. 300 µm auf, welche lichtmikroskopisch ermittelt wurde.

Um Hohlprofile mit noch kleineren Innendurchmessern herzustellen, wurden die Röhrchen fünf Tage bei 23 °C und einer relativen Luftfeuchtigkeit von 45% gelagert und anschließend verstreckt.

Zum Verstrecken wurden die Röhrchen an beiden Enden eingespannt und unter Einwirkung von heißem Wasserdampf erweicht. In diesem thermoplastischen Zustand wurden sie mit einem Verstreckungsverhältnis von ca. 1,4 gelängt, in diesem Zustand fixiert und während 16 Stunden bei 23 °C und einer relativen Luftfeuchtigkeit von 45% getrocknet.

Um die physiologische Abbauzeit der Röhrchen zu verlängern, können diese nach dem Verstrecken, wie in Beispiel 1 beschrieben, einer zweiten Vernetzung in der Gasphase unterzogen werden. Dabei können die Enden der Röhrchen verschlossen werden, so dass die Vernetzung nur von außen erfolgt.

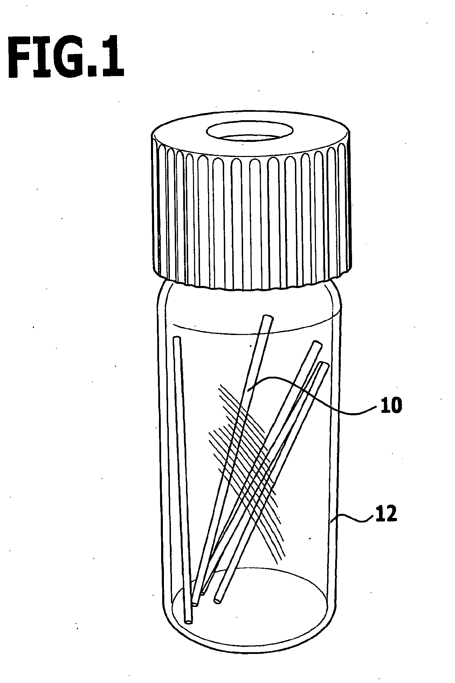

In der Figur 1 sind einige der auf diese Weise hergestellten Röhrchen 10 mit einer Länge von ca. 3 cm in einem Glasgefäß 12 dargestellt.

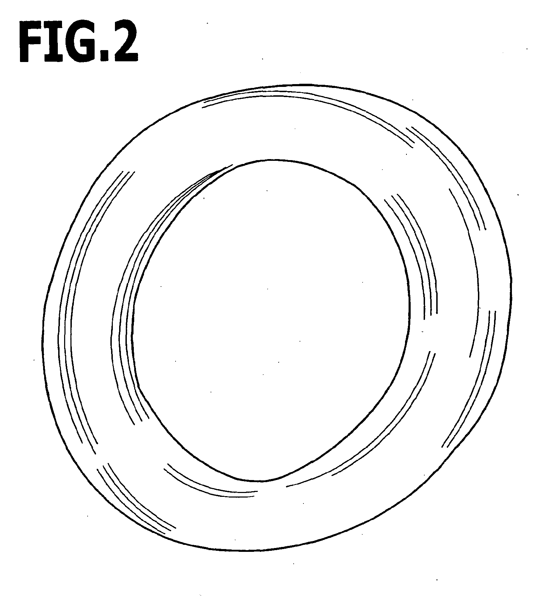

Figur 2 zeigt eine lichtmikroskopische Aufnahme des Querschnitts durch eines der verstreckten Röhrchen. Dieses weist einen Innendurchmesser von ca. 1.100 µm und eine Wandstärke von ca. 200 µm auf: Sowohl die Querschnittsform als auch die Wandstärke des Röhrchens sind äußerst regelmäßig.

Implantate mit diesen Abmessungen eignen sich besonders gut zur Verwendung als Nervenleitschienen. Auch eine stärkere Vernetzung der Röhrchen von der Außenseite her ist für diese Anwendung vorteilhaft, da hierdurch das Implantat im Zuge des Wachstums der Nervenzelle von innen heraus abgebaut werden kann.

Durch eine Erhöhung des Verstreckungsverhältnisses können auch erfindungsgemäße Implantate mit einem noch kleineren Innendurchmesser hergestellt werden, die für andere Anwendungen vorteilhaft sein können. Insbesondere ist es unter Anwendung des erfindungsgemäßen Verfahrens möglich, äußerst dünne Röhrchen mit einem Innendurchmesser im Bereich von 150 µm herzustellen, die als Drainage für das Auge eingesetzt werden können.

## Patentansprüche

1. Verfahren zur Herstellung eines Implantats in Form eines Hohlprofils auf Basis eines vernetzten, Gelatine enthaltenden Materials, wobei das Hohlprofil einen polygonalen Querschnitt aufweist und eine Wandung umfasst, welche ein Lumen umgibt, umfassend:
a) Herstellen einer wässrigen Lösung eines Gelatine enthaltenden Materials;
b) partielles Vernetzten des gelösten, Gelatine enthaltenden Materials;
c) Aufbringen der Lösung auf die Oberfläche eines das Lumen definierenden Formelements;
d) mindestens partielles Trocknenlassen der Lösung auf dem Formelement unter Ausbildung eines Hohlprofils auf Basis des vernetzten, Gelatine enthaltenden Materials; und
e) Vernetzen des in dem Hohlprofil enthaltenen Materials.

2. Verfahren nach Anspruch 1, wobei das Hohlprofil einen runden Querschnitt aufweist.

3. Verfahren nach Anspruch 1 oder 2, wobei c) mittels Eintauchen des Formelements in die Lösung erfolgt.

4. Verfahren nach einem der Ansprüche 1 bis 3, wobei c) und d) im Anschluss an d) ein- oder mehrmals wiederholt werden.

5. Verfahren nach einem der vorangehenden Ansprüche, wobei das Material einen Weichmacher umfasst.

6. Verfahren nach einem der vorangehenden Ansprüche, wobei die Gelatinekonzentration in der Lösung 5 bis 45 Gew.%, vorzugsweise 10 bis 30 Gew.%, beträgt.

7. Verfahren nach einem der vorangehenden Ansprüche, wobei die Vernetzung gemäß e) durch das Einwirken eines Vernetzungsmittels in der Gasphase durchgeführt wird.

8. Verfahren nach einem der vorangehenden Ansprüche, wobei das Hohlprofil im Anschluss an d) in Längsrichtung verstreckt wird.

9. Verfahren nach Anspruch 8, wobei das Hohlprofil unmittelbar vor dem Verstrecken durch eine Erhöhung der Temperatur und/oder des Wassergehalts in einen thermoplastischen Zustand überführt wird.

10. Verfahren nach Anspruch 8 oder 9, wobei das Verstrecken mit einem Verstreckungsverhältnis von 1,4 bis 8 durchgeführt wird.

11. Verfahren nach einem der Ansprüche 8 bis 10, wobei das Hohlprofil vor dem Verstrecken drei bis sieben Tage gelagert wird.

12. Verfahren nach einem der Ansprüche 8 bis 11, wobei die zweite Vernetzung (e) des Gelatine enthaltenden Materials nach dem Verstrecken des Hohlprofils durchgeführt wird.

13. Verfahren nach einem der vorangehenden Ansprüche, wobei in die Lösung gemäß a) ein Verstärkungsstoff zugegeben wird.

14. Implantat in Form eines Hohlprofils mit einem Innendurchmesser von 5 bis 30 mm, hergestellt gemäß dem Verfahren nach einem der vorangehenden Ansprüche, wobei das Implantat ein Stent für die Speiseröhre, für die Luftröhre oder für den Darm ist.

15. Implantat nach Anspruch 14, wobei das Gelatine enthaltende Material in einem inneren, dem Lumen benachbarten Bereich der Wandung einen anderen Vernetzungsgrad aufweist als in einem äußeren Bereich.

16. Implantat nach Anspruch 14 oder 15, wobei der Stent eine mittlere Wandstärke von 300 bis 1.500 µm aufweist.

## Claims

1. A method for producing an implant in the form of a hollow profile based on a cross-linked, gelatinous material, the hollow profile having a polygonal cross-section and comprising a wall, which surrounds a lumen, the method comprising:
a) preparing an aqueous solution of a gelatinous material;
b) partially cross-linking the dissolved, gelatinous material;
c) applying the solution to the surface of a shaped element which defines the lumen;
d) leaving the solution to dry at least partially on the shaped element, a hollow profile based on the cross-linked, gelatinous material being formed; and
e) cross-linking the material comprised in the hollow profile.

2. The method according to claim 1, the hollow profile having a round cross-section.

3. The method according to claim 1 or 2, step c) being effected by means of dipping the shaped element into the solution.

4. The method according to any of claims 1 to 3, steps c) and d) being repeated one or more times subsequent to step d).

5. The method according to any of the preceding claims, the material comprising a plasticizer.

6. The method according to any of the preceding claims, the concentration of gelatin in the solution being 5 to 45% by weight, preferably 10 to 30% by weight.

7. The method according to any of the preceding claims, the cross-linking in accordance with step e) being carried out by the action of a cross-linking agent in the gas phase.

8. The method according to any of the preceding claims, the hollow profile being stretched in the longitudinal direction subsequent to step d).

9. The method according to claim 8, the hollow profile being brought into a thermoplastic state directly before stretching, by raising temperature and/or water content.

10. The method according to claim 8 or 9, stretching being carried out with a stretch ratio of 1.4 to 8.

11. The method according to any of claims 8 to 10, the hollow profile being stored, before stretching, for three to seven days.

12. The method according to any of claims 8 to 11, the second cross-linking (step e)) of the gelatinous material being carried out after stretching of the hollow profile.

13. The method according to any of the preceding claims, a reinforcing material being added to the solution produced by step a).

14. An implant in the form of a hollow profile having an internal diameter of 5 to 30 mm, produced according to the method of any of the preceding claims, the implant being a stent for the esophagus, for the trachea or for the intestines.

15. The implant according to claim 14, the gelatinous material having a different degree of cross-linking in an inner region of the wall adjacent to the lumen than in an outer region.

16. The implant according to claim 14 or 15, the stent having an average wall thickness of 300 to 1,500 µm.

## Revendications

1. Procédé pour produire un implant sous la forme d'un profilé creux à base d'un matériau réticulé contenant de la gélatine, dans lequel le profilé creux présente une section polygonale et comprend une paroi qui entoure un lumen, comprenant :
a) préparation d'une solution aqueuse d'un matériau contenant de la gélatine ;
b) réticulation partielle du matériau en solution contenant de la gélatine ;
c) application de la solution sur la surface d'un élément de mise en forme définissant le lumen ;
d) séchage au moins partiel de la solution sur l'élément de mise en forme avec réalisation d'un profilé creux à base du matériau réticulé contenant de la gélatine ; et
e) réticulation du matériau contenu dans le profilé creux.

2. Procédé selon la revendication 1, dans lequel le profilé creux présente une section arrondie.

3. Procédé selon la revendication 1 ou 2, dans lequel l'étape c) a lieu par plongée de l'élément de mise en forme dans la solution.

4. Procédé selon l'une des revendications 1 à 3, dans lequel les étapes c) et d) sont répétées une ou plusieurs fois à la suite de l'étape d).

5. Procédé selon l'une des revendications précédentes, dans lequel le matériau comprend un plastifiant.

6. Procédé selon l'une des revendications précédentes, dans lequel la concentration en gélatine dans la solution est de 5 à 45 % en poids, de préférence 10 à 30 % en poids.

7. Procédé selon l'une des revendications précédentes, dans lequel la réticulation selon l'étape e) est effectuée par action d'un agent de réticulation dans la phase gazeuse.

8. Procédé selon l'une des revendications précédentes, dans lequel le profilé creux est étiré en direction longitudinale à la suite de l'étape d).

9. Procédé selon la revendication 8, dans lequel le profilé creux est transféré dans un état thermoplastique immédiatement avant l'étirage, par augmentation de la température et/ou de la teneur en eau.

10. Procédé selon la revendication 8 ou 9, dans lequel l'étirage est exécuté avec un rapport d'étirage de 1,4 à 8.

11. Procédé selon l'une des revendications 8 à 10, dans lequel le profilé creux est stocké pendant trois à sept jours avant l'étirage.

12. Procédé selon l'une des revendications 8 à 11, dans lequel la seconde réticulation (e) du matériau contenant de la gélatine est effectuée après l'étirage du profilé creux.

13. Procédé selon l'une des revendications précédentes, dans lequel un agent de renforcement est ajouté dans la solution selon l'étape a).

14. Implant sous la forme d'un profilé creux avec un diamètre intérieur de 5 à 30 mm, produit selon le procédé de l'une des revendications précédentes, dans lequel l'implant est un stent pour l'oesophage, pour la trachée-artère ou pour l'intestin.

15. Implant selon la revendication 14, dans lequel le matériau contenant de la gélatine présente dans une région intérieure de la paroi proche du lumen un degré de réticulation autre que dans une région extérieure.

16. Implant selon la revendication 14 ou 15, dans lequel le stent présente une épaisseur de paroi moyenne de 300 à 1500 µm.
